# EUROPÄISCHE PATENTSCHRIFT

(11) **EP 1 648 432 B1**
(45) Veröffentlichungstag und Bekanntmachung des Hinweises auf die Patenterteilung: **25.08.2010**
(21) Anmeldenummer: 04741204.4
(22) Anmeldetag: 22.07.2004
(51) Int. Cl.: A61K 31/135, A61K 31/381

(54) **VERWENDUNG VON ROTIGOTIN ZUR BEHANDLUNG VON DEPRESSIONEN**
USE OF ROTIGOTINE FOR THE TREATMENT OF DEPRESSION
UTILISATION DE ROTIGOTINE POUR LE TRAITEMENT DES DEPRESSIONS

(30) Priorität: 26.07.2003 DE 10334188
(43) Veröffentlichungstag der Anmeldung: 26.04.2006
(73) Patentinhaber: UCB Pharma GmbH, 40789 Monheim (DE)
(72) Erfinder: SCHELLER, Dieter, 41470 Neuss (DE); BREIDENBACH, Alexander, 79576 Weil am Rhein (DE); SELVE, Norma, 53842 Troisdorf (DE)
(74) Vertreter: HOFFMANN EITLE
(86) Internationale Anmeldenummer: PCT/EP2004/008168
(87) Internationale Veröffentlichungsnummer: WO 2005/009424

(56) Entgegenhaltungen:
- WO-A1-2004/058247
- WO-A1-2005/009425
- US-A1- 2003 026 830
- VERHAGEN METMAN L ET AL: "CONTINUOUS TRANSDERMAL DOPAMINERGIC STIMULATION IN ADVANCED PARKINSON'S DISEASE" CLINICAL NEUROPHARMACOLOGY, RAVEN PRESS, NEW YORK, NY, US, Bd. 24, Nr. 3, Mai 2001 (2001-05), Seiten 163-169, XP008020182 ISSN: 0362-5664

## Beschreibung

Nach Schätzungen der WHO wird die Depression bis 2020 die zweithäufigste Ursache für erkrankungsbedingte Behinderung sein (Murray, Lancet 349 (1997) 1498). Die Effizienz gegenwärtiger pharmakologischer Behandlungen ist aus verschiedenen Gründen, z.B. wegen spätem Wirkeintritt, Nebenwirkungen oder fehlender Wirksamkeit der Arzneimittel begrenzt. Aufgrund der Häufigkeit und Dauer dieser Erkrankung und der Rezidivneigung besteht ein großer Bedarf an neuen, innovativen Antidepressiva.

Bisher werden als Antidepressiva vorwiegend Amin-Wiederaufnahmehemmer oder Monoaminoxidase-Inhibitoren eingesetzt (Goodman & Gilman's, The pharmacological basis of therapeutics, 9th Edition). In jüngster Zeit wird als vielversprechendes Therapiekonzept die Verwendung von Wirkstoffen diskutiert, die sowohl serotonerge (5HT1) als auch adrenerge Rezeptoren (a2) beeinflussen (Westenberg, J. Clin. Psychiatry 60, Suppl. 17, 1999, 4; Schatzberg, Human Psychopharmacology 17, 2002, S. 17). Ein Beispiel für einen Wirkstoff mit solch einem dualen Wirkprinzip ist das Mirtazapin (Gorman, J. Clin. Psychiatry 60, Suppl. 17, 1999, 9).

Von Wirkstoffen mit dualem Wirkprinzip wird ein rascherer Wirkeintritt und eine überlegene Wirksamkeit im Vergleich mit klassischen Antidepressiva erwartet, da die hohe Selektivität der Wirkstoffe und das damit verbundene günstige Nebenwirkungsprofil eine rasche Einstellung des Patienten auf die individuelle Erhaltungsdosis erlaubt (Deakin, Int. Clin. Psychopharmacology 17, Suppl. 1, 2002, S. 13).

Neuerlich wurde auch den Dopamin-Agonisten Pramipexol und Ropinirol eine antidepressive Wirksamkeit zugeschrieben und diese Wirkung in klinischen Studien belegt (Ostow, M., Am J Psychiatry. 2002 Feb;159(2):320-1). Dabei ist jedoch noch unklar, welchen Beitrag der Dopamin-Agonismus und welchen Beitrag mögliche andere Wirkungen der untersuchten Dopamin-Agonisten leisten, da diese substanzspezifisch auch andere Neurotransmittersysteme beeinflussen.

Es wurde nun überraschend gefunden, dass das als Dopamin-Agonist beschriebene und zur Behandlung von Morbus Parkinson in Form eines transdermalen therapeutischen Systems eingesetzte Rotigotin (US 2003/0026830 und Metman et al., Clinical Neuropharmacol. 24, 2001, 163) sowohl an α2-Rezeptoren als auch an den 5HT1A-Rezeptor bindet. Während Rotigotin an α 2-Rezeptoren antagonistisch wirkt, zeigt es an 5HT1A-Rezeptoren agonistische Aktivität.

Mit diesem Profil, insbesondere im Hinblick auf die überraschende agonistische 5HT1A-Aktivität, ist Rotigotin [(-)-5,6,7,8-Tetrahydro-6-[propyl[2-(2-thienyl)ethyl]amino]-1-naphthol] ein Kandidat zur Verwendung als Antidepressivum.

In drei verschiedenen, validierten Tiermodellen wurde die Eignung von Rotigotin als Antidepressivum demonstriert.

Der "forced swim test" ist ein Tiermodell, bei dem depressive Episoden durch akuten Stress ausgelöst werden. Dabei werden Ratten in einem begrenzten Raum zum Schwimmen gezwungen. Nach initialen Selbstrettungsversuchen, in denen die Tiere die Ausweglosigkeit erfassen, verfallen sie in Bewegungslosigkeit. Bei einer Wiederholung des Versuchs verharren die Tiere von Beginn des Versuchs an in Bewegungslosigkeit. Bei Vorbehandlung mit Antidepressiva wird die Zeit der Bewegungslosigkeit beim Wiederholungsversuch verkürzt, die Tiere beginnen meist unmittelbar nach Transfer in das Wasserbecken mit Such- und Fluchtbewegungen (Porsolt, Biomedicine 30,1979, 139). Rotigotin führt zu einer deutlich verkürzten Immobilitätszeit.

Im "learned helplessness test" werden Ratten mehrfach unkontrollierbarem Stress ausgesetzt. Dies bewirkt bei den Tieren eine verschlechterte Lernfähigkeit in einer späteren Situation (z. B. nach 48 h), in der sie dem Stress wieder ausweichen könnten. Nach subchronischer, aber nicht akuter Gabe von Antidepressiva normalisiert sich die Lernfähigkeit wieder und die Tiere lernen, dem (angekündigten) Stress (rechtzeitige) zu entfliehen, (Sherman, Pharmacology Biochemistry & Behavior 16,1982, 449). Nach mehrtägiger Verabreichung von Rotigotindepotsuspension (Ausführungsbeispiel 2) zeigten .die Tiere bei niedrigen Konzentrationen ein verbessertes Lernverhalten; allerdings steigerten die höheren Dosen auch die Aktivität der Tiere unter Nicht-TestBedingungen.

In einem weiteren Tiermodell (Ausführungsbeispiel 3) wurde untersucht, ob die antidepressiven Wirkungen von Rotigotin von einer allgemeinen motorischen Stimulation unterschieden werden können. Hierbei wurde Rotigotin an Ratten verabreicht, deren Riechkolben beidseitig entfernt wurden. Die Entfernung der Riechkolben führt in der unbehandelten Kontroll-Gruppe zu einer adaptiven Hyperaktivität. Es ist aus der Literatur bekannt, dass chronisch verabreichte Antidepressiva in diesem Modell zu einer Reduktion der Bewegungsaktivität der Tiere führen, während Stimulantien die motorische Aktivität weiter steigern (van Riezen H et al, Br J Pharmacol. 60(4), 1977, 521; Kelly JP et al, Pharmacol Ther. 74(3), 1997, 299). Mit diesem Modell kann somit zwischen antidepressiven und unspezifisch-stimulatorischen Effekten eines Wirkstoffs diskriminiert werden. Es zeigte sich, dass Rotigotin niedrig dosiert eine spezifisch antidepressive Wirkung zeigt, die in etwa der Wirkung des Antidepressivums Imipramin entspricht und die zur nahezu vollständigen Unterdrückung der Bulbektomie-induzierten lokomotorischen Hyperaktivität führt. Bei höheren Rotigotinkonzentrationen überwiegt hingegen der stimulatorische dopamin-agonistische Effekt.

Damit konnte klar gezeigt werden, dass subkutan appliziertes Rotigotin in allen drei Tests überraschenderweise eine signifikante antidepressive Wirkung hat.

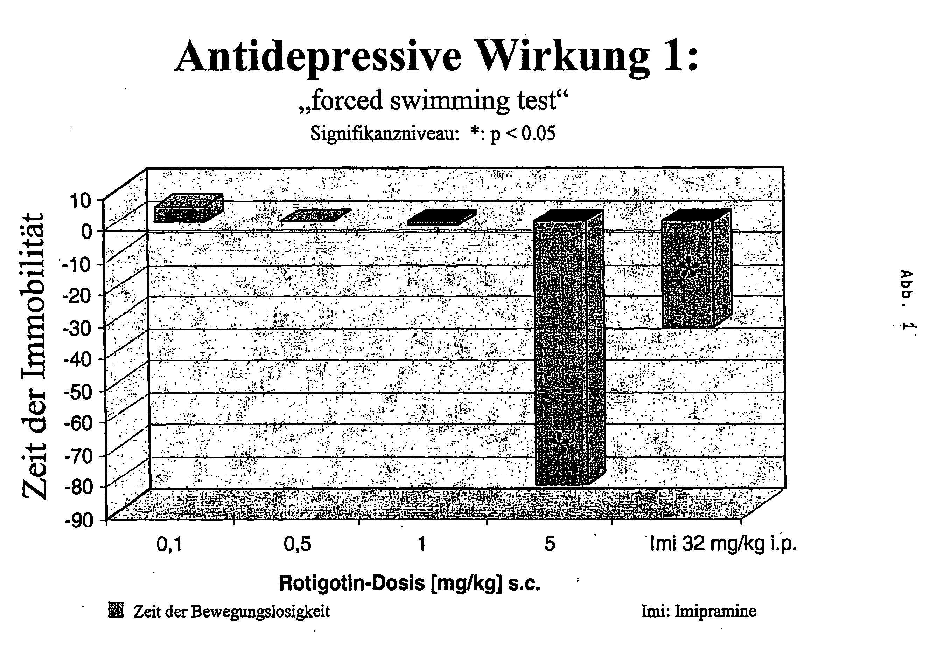

Abbildung 1 zeigt, dass Rotigotin im "forced swim test" zu einer deutlichen Reduktion der Immobilitätszeit führt.

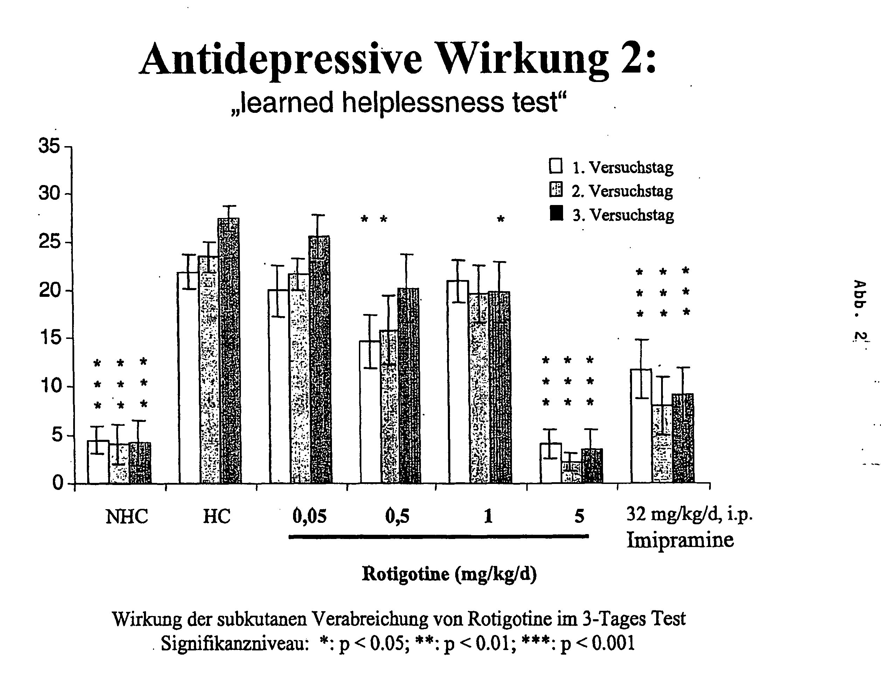

Abbildung 2 zeigt, dass mit Rotigotindepotsuspension (Ausführungsbeispiel 2) behandelte Tiere im "learned helplessness test" dosisabhängig ein normalisiertes Lernverhalten (NHC) gegenüber der nur mit Vehikel behandelten Kontrollgruppe (HC) zeigen.

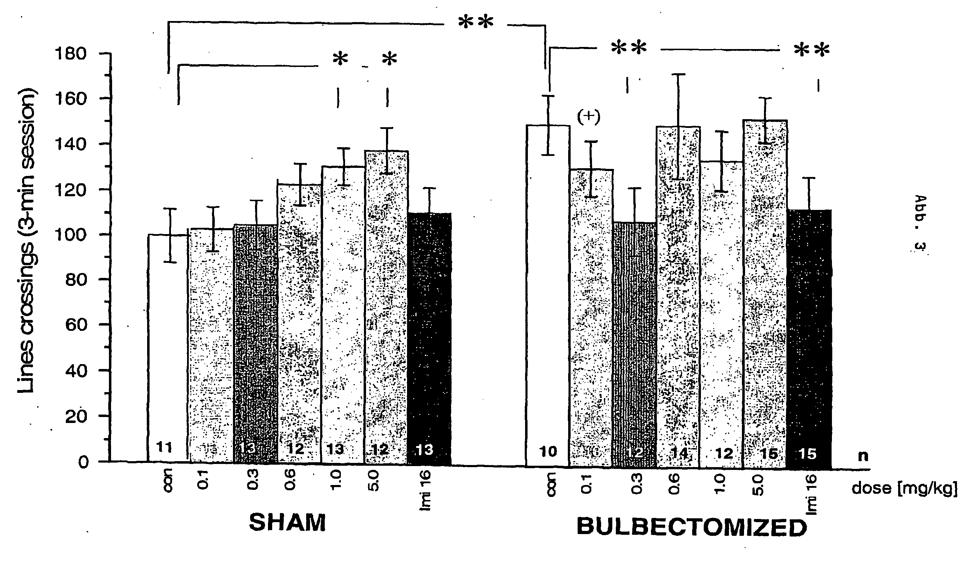

Abbildung 3 zeigt, dass Rotigotin in niedrigen Dosierungen in bulbectomisierten Ratten (Ausführungsbeispiel 3) die motorische Hyperaktivität deutlich reduziert und damit eine klare antidepressive Wirkung entfaltet. In höheren Dosierungen hingegen dominiert eine unspezifische Aktivierung der lokomotorischen Aktivität und tritt sowohl bei bulbectomisierten Tieren als auch bei Kontrolltieren auf.

Aus diesen präklinischen Daten ergibt sich die Schlussfolgerung, dass mit Rotigotin, seinen biologisch aktiven Metaboliten sowie den entsprechenden Prodrugs und Salzen neue wirksame Arzneimittel zur Behandlung von Depressionen zur Verfügung gestellt werden können.

Ein Gegenstand der Erfindung ist daher die Verwendung von Rotigotin, seiner Prodrugs und Salze zur Herstellung eines Arzneimittels zur Behandlung von Depressionen.
Der Begriff "Behandlung" umfasst in dieser Patentanmeldung sowohl die Behandlung bestehender Depressionen als auch die vorbeugende Behandlung (Prophylaxe) von Depressionen, z.B. von rezidivierenden depressiven Phasen.

Depressive Störungen werden zum besseren Verständnis und zur Erzielung einer optimalen individuellen Therapie in Unterformen unterteilt, wobei die Übergänge der verschiedenen Unterformen oft fließend sind. Die Klassifizierung der Depression erfolgt - traditionell - nach ihren vermeintlichen Ursachen oder - neuerdings - nach ihren Symptomen (siehe hierzu ICD-10 "International Statistical Classification of Diseases and Related Health Problems" der WHO).

Unter dem Begriff "Depression" werden in dieser Patentanmeldung sowohl die verschiedenen, unten genannten traditionellen Unterformen der Depression verstanden, als auch die im ICD-10 unter dem Begriff "affektiven Störungen" subsumierten Störungen, die mit depressiven Episoden einhergehen, insbesondere depressive Episoden, rezidivierende depressive Störungen, depressive Phasen bei bipolaren affektiven Störungen sowie Angststörungen, Anpassungsstörungen und hirnorganische Erkrankungen, die jeweils mit depressiven Symptomen einhergehen. Entsprechende Störungen sind beispielsweise in den ICD-10 Klassifikationen (Version 2.0, November 2000) F31, F32, F33, F41, F43, F45 und F06 aufgeführt.

Bei der traditionellen Unterteilung der Depression nach Ursachen werden üblicherweise 4 Hauptklassen unterschieden:
I. Endogene Depressionen
   Bei endogener Depression lassen sich keine ohne weiteres erkennbaren äußeren Ursachen als Auslöser der Depression identifizieren. Auslöser sind wahrscheinlich Störungen des Neurotransmittersystems des Gehirns. Typisch für endogene Depressionen ist der phasenhafte Verlauf, wobei die depressiven Episoden wiederholt auftreten können. Endogene Depressionen werden in der Regel unterteilt in
   - unipolare Depressionen ("major depression"), bei der nur depressive Phasen auftreten.
   - bipolare Depressionen ("manisch-depressive Störungen"), bei denen depressive Episoden mit manischen Phasen wechseln.
II. Somatogene Depressionen
   Ursache dieser Depressionen sind körperlich-organische Störungen. Im Allgemeinen werden somatogene Depressionen unterteilt in
   - organische Depressionen, die auf einer Erkrankung oder Verletzung des Gehirns beruhen. Solche Erkrankungen oder Verletzungen, die häufig mit einem veränderten Hirnstoffwechsel einhergehen, sind z.B. Hirntumore, Morbus Parkinson, Migräne, Epilepsie, Hirnlähmung, Hirnarteriosklerose, Hirntraumen, Hirnhautentzündung, Schlaganfall und Demenzen, wie z.B. die Alzheimersche Erkrankung;
   - symptomatische Depression, die oft als Folge oder Begleiterscheinung einer Krankheit aufritt, die die Himfunktion nur indirekt beeinflusst. Dies kann z.B. eine Kreislauferkrankung, Hypothyreose oder eine andere Hormonstörung, Infektionskrankheit, Krebs oder Lebererkrankung sein;
   - pharmakogene Depression, z.B. bei Alkohol-, Medikamenten- oder Drogenmissbrauch.
III. Psychogene Depressionen
   Diese sind oft Überreaktionen auf ein oder mehrere traumatische Erlebnisse. Die Unterteilung erfolgt häufig in Erschöpfungs-Depression, neurotische Depression und reaktive Depression auf Grund aktueller Konflikte oder Ereignisse.
IV. Depressionen in besonderen Lebenslagen
   Beispiele sind Wochenbett-Depressionen, Alters-Depressionen, Depressionen im Kindesalter, saisonale Depressionen sowie Pubertätsdepressionen.

Rotigotin sowie dessen Prodrugs gemäß Anspruch 1 und Salze sind grundsätzlich für die i-erstellung eines Medikaments zur Behandlung der verschiedenen, oben genannten Depressionsformen bzw. zur Behandlung von affektiven Störungen, insbesondere von depressiven Episoden, rezidivierenden depressiven Störungen und von depressiven Phasen bei bipolaren affektiven Störungen, entsprechend der ICD-10 geeignet.

Erfindungsgemäß wird Rotigotin bevorzugt zur Herstellung eines Medikaments zur Behandlung depressiver Episoden und schwerer rezidivierender depressiver Störungen verwendet, wie sie beispielsweise bei der endogenen, unipolaren Depression ("major depression") auftreten.

Als Ursachen für endogene, unipolare Depressionen werden Stoffwechselstörungen der Gehirnzellen, d.h. Noradrenalin- oder Serotoninmangel und/oder eine genetische Prädisposition angesehen.

Unter dem Begriff "major depression" wird in dieser Patentanmeldung insbesondere eine Störung bezeichnet, wie im amerikanischen Diagnose-Manual "The Diagnostic and Statistic Manual of Mental Disorders - 4th Edition" (American Psychiatric Association. 1994; "DSM IV") beschrieben.

Rotigotin [(-)-5,6,7,8-Tetrahydro-6-[propyl[2-(2-thienyl)ethyl]amino]-1-naphthol] und seine Prodrugs und Salze sind auch besonders geeignet zur Herstellung von Antidepressiva zur Behandlung depressiver Episoden bei manisch-depressiven Patienten. Diese depressiven Phasen bei bipolaren Störungen werden in dieser Patentanmeldung unter dem Begriff "Depressionen" subsumiert.

Ferner wird Rotigotin bevorzugt zur Herstellung eines Arzneimittels zur Behandlung "organischer" Depressionen verwendet, wie weiter oben beschrieben. Organische Depressionen treten beispielsweise häufig bei Parkinson-Erkrankungen, bzw. bei zerebrovaskulären Erkrankungen und bei dementiellen Störungen auf.

Bei der Behandlung von Depressionen, die ais Folge von Morbus Parkinson auftraten, ergibt sich aus der vorliegenden Erfindung die für die klinische Praxis relevante Schlussfolgerung, dass die übliche Komedikation von Antidepressiva und Antiparkinson-Mitteln nicht erforderlich ist, wenn die depressive Parkinson-Patienten auf Rotigotin eingestellt werden.

Ein Gegenstand der Erfindung ist daher die Verwendung von Rotigotin, seiner Prodrugs gemäß Anspruch 1 und Salze, zur Herstellung eines Arzneimittels zur Behandlung von mit Morbus Parkinson verbundenen Depressionen, wobei auf eine Komedikation mit anderen Antidepressiva optional verzichtet werden kann.

Ein anderer Gegenstand der Erfindung ist die Verwendung von Rotigotin, seiner Prodrugs gemäß Anspruch 1 und Salze, jeweils alleine oder in Kombination mit anderen Antidepressiva, zur Behandlung organischer Depressionen, die nicht im Zusammenhang mit Morbus Parkinson stehen. Beispiele für solche organischen Depressionen sind Depressionen im Zusammenhang mit Himtumoren, Migräne, Epilepsie, Hirnlähmung, Hirnarteriosklerose, Hirntraumen, Hirnhautentzündung, Schlaganfall, Demenz, Alzheimer'sche Erkrankung oder dem Parkinson Plus Syndrom.

Ein weiterer Gegenstand der Erfindung ist eine Methode zur Behandlung einer Depression bei einem Säuger, insbesondere einer endogenen, unipolaren Depression ("major depression"), einer depressiven Phase einer bipolaren Störung, einer Parkinsonassoziierten Depression oder einer von Morbus Parkinson unabhängigen organischen Depression durch Verabreichung einer therapeutisch wirksamen Menge von Rotigotin, eines Metaboliten, Prodrugs oder Salzes an besagten Säuger, insbesondere an einen Menschen.

Unter Prodrugs gemäß Anspruch 1 von Rotigotin werden in dieser Patentanmeldung insbesondere Verwindungen verstanden, die im menschlichen Körper, insbesondere im Plasma oder beim Durchtritt durch Haut oder Schleimhaut in therapeutisch effektiver Menge zu Rotigotin gespalten, umgesetzt oder metabolisiert werden.

Diese Prodrugs sind Ester, insbesondere Alkanoylester und besonders bevorzugt Alkanoylester mit bis zu 6 Kohlenstoffatomen, Carbamate, Carbonate, Ketal, Acetal, Phosphate, Phosphonate, Sulfate und Sulfonate.

Die Herstellung der Prodrugs gemäß Anspruch 1 durch Reaktion von Rotigotin mit entsprechend reaktiven Vorstufen wie Säurechloriden, Säureanhydriden, Carbamoylchloriden, Sufonylchloriden etc. ist dem Fachmann auf dem Gebiet der medizinischen Chemie bekannt und lässt sich der einschlägigen Fachliteratur entnehmen.

Beispiele für Literaturstellen sind Bundgaard: Design of Prodrugs, Elsevier, Amsterdam, 1985; Higuchi und Stella: Pro-drugs as novel drug delivery systems in American Chemical Society, Washington DC, 1975; Sloan: Prodrugs - Topical and Ocular Drug Delivery, Ed: M. Dekker, 1992; Roche: Design of biopharmaceutical properties through prodrugs and analogs, Washington, DC, 1977.

Die grundsätzliche Eignung eines Rotigotin-Derivats als Prodrug kann bestimmt werden, indem die jeweilige Verbindung unter definierten Bedingungen-mit einer Enzymmischung, einer Zellpräparation, einem Zellhomogenisat oder einer enzymhaltigen Zellfraktion inkubiert wird und das entstehende Rotigotin gemessen wird. Eine geeignete Enzymmischung ist beispielsweise enthalten in der S 9-Leberpräparation der Firma Gentest, Woburn, MA, USA. Zur Messung besonders schnell spaltbarer Prodrugs kann das zu testende Prodrug auch in Plasma, z.B. Plasma aus Humanblut, inkubiert werden. Die optimale Hydrolysegeschwindigkeit des Prodrugs hängt von der Zielsetzung ab. Schnell spaltbare Prodrugs können beispielsweise zur raschen Anflutung, z.B. bei nasaler Verabreichung, geeignet sein. Langsamer spaltbare Prodrugs können beispielsweise zur Retardierung, z.B. bei transdermaler, parenteraler oder oraler Gabe geeignet sein.

Verschiedene Prodrugs des Racemats von Rotigotin (N-0437) sind beispielsweise beschrieben in Den Haas et al., Naunyn-Schmiedeberg's Arch Pharmacol 342, 1990, 655 und Den Haas et al, J. Pharm Pharmacol 43, 1991, 11.

In-vivo sollte ein Prodrug soviel Rotigotin freisetzen, dass eine therapeutisch effektive steady-state Konzentration Rotigotin im Plasma erreicht wird. Als therapeutisch effektive Konzentrationen werden dabei im Allgemeinen Rotigotinkonzentrationen zwischen 0.05 und 20 ng/mL, bevorzugt zwischen 0.1 ng und 10 ng/mL und besonders bevorzugt zwischen 0.2 und 5 ng/mL Plasma angesehen.

Zur spezifischen Behandlung von Depressionen können gegebenenfalls aber auch niedrigere Rotigotin-Plasmaspiegel ausreichend sein, z.B. solche unter 2 ng/ml, z.B. zwischen 0,05 und 1 ng/ml Plasma oder zwischen 0,1 und 0,5 ng/ml Plasma.

Rotigotin ist das S-(-)-Enantiomer von 5,6,7,8-Tetrahydro-6-[propyl[2-(2-thienyl)ethyl]amino]-1-naphthol. Dies bedeutet, dass der Anteil des (R)-Enantiomeren im Arzneimittel erfindungsgemäß gering ist. Das (R)-Enantiomer liegt bevorzugt mit einem Anteil von < 10 Mol%, besonders bevorzugt mit einem Anteil von < 2 Mol% und ganz besonders bevorzugt mit einem Molanteil von < 1 %, bezogen auf die Gesamtmenge Rotigotin, im Antidepressivum vor.

Rotigotin und seine Prodrugs gemäß Anspruch 1 können als freie Basen oder in Form der physiologisch akzeptablen Salze, z.B. in Form des Hydrochlorids, im Arzneimittel vorliegen.

"Physiologisch akzeptable Salze" schließen nicht-toxische Additionssalze einer Base, insbesondere einer Verbindung der Formel (I) in Form der freien Base, mit organischen oder anorganischen Säuren, z.B. mit HCl, ein.

Zur Verabreichung von Rotigotin und seinen Prodrugs gemäß Anspruch 1 stehen verschiedene Applikationswege zur Verfügung, die der Fachmann je nach Bedarf, Zustand und Alter des Patienten, erforderlicher Dosierung und gewünschtem Applikationsintervall auswählen und anpassen kann.

Eine bevorzugte Art der Verabreichung von Rotigotin ist die transdermale Gabe. Die Darreichungsform kann grundsätzlich ausgewählt sein aus z.B. Salbe, Paste, Spray, Folie, Pfiaster oder einer iontophoretischen Vorrichtung.

Bevorzugt wird Rotigotin dabei in Pflasterform auf die Haut des Patienten gebracht, wobei der Wirkstoff bevorzugt in einer Matrix aus adhesivem Polymer, z.B. einem selbstklebenden adhesiven Polysiloxan, vorliegt (Ausführungsbeispiel 1). Beispiele für geeignete transdermale Formulierungen finden sich in WO 99/49852, WO 02/89777 und WO 02/89778. Eine solche Darreichungsform ermöglicht die Einstellung eines weitgehend konstanten Plasmaspiegels und damit eine konstante dopaminerge Stimulation über das gesamte Applikationsintervall (WO 02/89778; Metman, Clinical Neuropharmacol. 24, 2001, 163).

Wird dagegen ein Antidepressivum in Form einer subkutanen oder intramuskulären Depotform gewünscht, kann das Rotigotin beispielsweise ais Salzkristall, z.B. als kristallines Hydrochlorid, in einem hydrophoben, wasserfreien Medium suspendiert und injiziert werden, wie in WO 02/15903 beschrieben oder auch in Form von Mikrokapseln, Mikropartikeln oder Implantaten auf Basis bioabbaubarer Polymere, wie beispielsweise in WO 02/38646 beschrieben, verabreicht werden.

Andere denkbare Formen der Verabreichung von Rotigotin und seinen Prodrugs gemäß Anspruch 1 sind transmukosale Formulierungen, z.B. Sublingualsprays, nasale oder rektale Formulierungen oder Aerosole zur pulmonalen Verabreichung.

Geeignete Dosierungen von Rotigotin liegen zwischen 0,1 und ca. 50 mg/Tag, wobei vorzugsweise Tagesdosen zwischen 0,2 und 40 mg und insbesondere zwischen 0,4 und 20 mg/Tag verabreicht werden. Besonders bevorzugte Dosierungen von Rotigotin liegen oberhalb von 0,5 mg/Tag, wobei für Rotigotin-Anwendungen, die keine gleichzeitige Behandlung von motorischen Störungen von Morbus Parkinson erfordern, ganz besonders bevorzugt solche Dosierungsformen ausgewählt werden, in denen die antidepressive Wirkung von Rotigotin ausgeprägt ist, bei denen die unspezifisch stimulatorische Wirkung von Rotigotin aber möglichst gering ist. Solche Dosierungen liegen im Allgemeinen unter 10 mg/Tag, z.B. unter 7,5 mg oder unter 5, 4, 3, 2 oder unter 1 mg/Tag und insbesondere zwischen 0,5 und 5 mg/Tag.

Bei Morbus Parkinson-Patienten kann dagegen eine Dosierung von z.T. oberhalb von 5 mg/Tag zur gleichzeitigen Therapie der motorischen Störungen erforderlich sein. Entsprechende Dosierungen liegen z.B. in Abhängigkeit von Alter und Verfassung des Patienten, Schweregrad der Erkrankung etc. zum Teil bei deutlich über 1 mg/Tag, z.B. bei über 5, 6, 7, 8, 9, 10 oder sogar zwischen 10 und 50 mg/Tag, z.B. zwischen 10 und 25 mg/Tag.

In Abhängigkeit von der gewählten Applikationsart kann die gewünschte Tagesdosis durch das Formulierungsdesign gesteuert werden. Beispielsweise kann die Tagesdosis von transdermal verabreichtem Rotigotin durch die Einstellung einer entsprechenden Fluxrate pro flächeneinheit und/oder durch Variation der Pflastergröße eingestellt werden. Dabei kann die Dosierung einschleichend erfolgen, das heißt, die Behandlung kann gegebenenfalls mit niedrigen Dosierungen beginnen, die dann bis zur Erhaltungsdosis gesteigert werden.

Ein Gegenstand der Erfindung ist daher eine Dosierungsform, z.B. ein Pflaster oder eine injizierbare Depotformulierung, die die entsprechende zur Therapie der Depression erforderliche Menge Rotigotin, z.B. zwischen 0,5 und 10 mg/Tag oder zwischen 0,5 und 5 mg/Tag, wie weiter oben beschrieben, freisetzt.

Dem Fachmann ist klar, dass das Dosierungsintervall in Abhängigkeit von der applizierten Menge, der Applikationsart und dem Tagesbedart des Patienten variieren kann. So kann eine transdermale Applikationsform beispielsweise zur einmal täglichen, dreitägigen oder siebentägigen Verabreichung konzipert sein, während ein subkutanes oder intramuskuläres Depot Injektionen beispielsweise im Ein-, Zwei- oder Vierwochen-Rhythmus ermöglichen kann.

Rotigotin und seine Prodrugs gemäß Ansprunch 1 können als Monotherapeutika zur Behandlung der Depression eingesetzt werden. In einer Ausführungsform der Erfindung können in der antidepressiven Arzneiform neben Rotigotin aber auch noch andere Wirkstoffe vorliegen.

Beispiele hierfür sind andere Antidepressiva, die den Serotonin- oder Noradrenalin-Stoffwechsel direkt oder indirekt beeinflussen.
Beispiele hierfür sind
- selektive Serotonin-Wiederaumahmenemmer wie Sertralin, Citalopram, Paroxetin oder Fluoxetin
- gemischte Serotonin-, Noradrenalin-Wiederaufnahmehemmer wie Venlaxafin, Milnacipram, Mirtazapin und trizyklische Antidepressiva wie Amitryptilin und imipramin
- selektive Noradrenalin-Wiederaufnahmehemmer wie Reboxetin
- Monoaminoxidase-Hemmer wie Tranylcypramin oder Clorgylin
- Alpha2-Rezeptor und/oder Serotoninrezeptor-Modulatoren wie Mirtazapin oder Nefazodon.

Andere Beispiele für Antidepressiva sind Adenosin-Antagonisten, wie z.B. ST 1535, Sigma-Opioidrezeptor-Liganden, NK-Antagonisten wie GW 597599, Saredudant oder Aprepitant, Melatonin-Agonisten oder Modulatoren der Hypothalamus-Hypophyse-Nebennieren-Achse.

In Abhängigkeit von der Ursache und den Symptomen der Depression kann ein Kombinationspräparat auch ein zusätzliches Antipsychotikum, Sedativum, Anxiolytikum oder Migränemittel, bzw. einen Wirkstoff enthalten, der ein oder mehrere Wirkungen ausgewählt aus antidepressiver, antipsychotischer, sedativer, anxiolytischer oder antimigränoider Wirkung entfaltet.

Dabei können die Verbindung der Formel I oder II und das zusätzliche Antidepressivum, Antipsychotikum, Sedativum, Anxiolytikum oder Migränemittel in der gleichen pharmazeutischen Formulierung, z.B. einer Kombinationstablette, oder auch in unterschiedlichen Applikationseinheiten vorliegen, z.B. in Form zweier separater Tabletten. Je nach Bedarf können beide Wirkstoffe gleichzeitig oder zeitlich getrennt verabreicht werden.

In einem Kombinationspräparat kann eine sequentielle Gabe beispielsweise erreicht werden, indem eine Darreichungsform, z.B. eine orale Tablette, zwei unterschiedliche Schichten mit differierendem Freisetzungsprofil für die verschiedenen pharmazeutisch aktiven Bestandteile aufweist. Dem Fachmann ist klar, dass im Kontext der vorliegenden Erfindung verschiedene Darreichungsformen und Applikationsschemata denkbar sind, die alle Gegenstand der Erfindung sind.

Beispiele für Antipsychotika sind Promethazin, Fluphenazin, Perphenacin, Levomepromazin, Thioridazin, Perazin, Promazin, Chlorprothixen, Zuclopenthixol, Prothipendyl, Flupentixol, Zotepin, Benperidol, Pipamperon, Melperon, Haloperidol, Bromperidol, Sulpirid, Clozapin, Pimozid, Risperidon, Quetiapin, Amisulprid, Olanzapin.

Beispiele für Sedativa sind Diphenhydramin, Doxylaminsuccinat, Nitrazepam, Midazolam, Lormetazepam, Flunitrazepam, Flurazepam, Oxazepam, Bromazepam, Triazolam, Brotizolam, Temazepam, Chloralhydrat, Zopiclon, Zolpidem, Tryptophan, Zaleplon.

Beispiele für Anxiolytika sind Fluspirilen, Thioridazin, Oxazepam, Alprazolam, Bromazepam, Lorazepam, Prazepam, Diazepam, Clobazam, Medazepam, Chlordiazepoxid, Dikaliumchlorazepat, Nordazepam, Meprobamat, Buspiron, Kavain, Hydroxyzin.

Beispiele für Migränemittel sind Almotriptan, Zolmitriptan, Acetylsalicylsäure, Ergotamin, Dihydroergotamin, Methysergid, Iprazochrom, Ibuprofen, Sumatriptan, Rizatriptan, Naratriptan, Paracetamol.

### Ausführungsbeispiele:

### Ausführungsbeispiel 1: Rotigotin-Pflaster

1.8 g Rotigotin (freie Base) werden in 2.4 g Ethanol gelöst und zu 0.4 g Kollidon 90F (gelöst in 1 g Ethanol) gegeben. Diese Mischung wird zu einer 74%igen Lösung von Silikonpolymeren (8.9 g BioPSA 7-4201 + 8.9 g BIO-PSA 7-4301 [Dow Corning]) in Heptan gegeben. Nach Zugabe von 2.65 g Petrolether wird die Mischung für 1 Stunde bei 700 UpM gerührt, um eine homogene Dispersion zu erhalten. Nach Laminierung auf Polyester wurde bei 50°C getrocknet. Das Pflastergewicht betrug schließlich 50 g/cm2.

### Ausführungsbeispiel 2: Rotigotin-Depotsuspensionen

(a) 1411,2 g Miglyol 812 wurde in eine Duran Flasche eingewogen. 14,4 g Imwitor 312 wurde dem Miglyol zugegeben und im Anschluß für 30 Minuten unter Rühren auf 80°C erwärmt. Die klare Lösung wurde auf Raumtemperatur abgekühlt und gefiltert.
(b) 1188 g der unter (a) hergestellten Lösung wurde in einen Glaslaborreaktor überführt, 12 g Rotigotin zugesetzt und für 10 Minuten mit einem Ultraturrax bei 10.000 UpM unter Stickstoff homogenisiert. Die Suspension wurde bei laufendem Ultraturrax (2.000 UpM) in Braunglasflaschen abgefüllt.

### Ausführungsbeispiel 3:

Die Bulbektomie-Studie wurde an Sprague-Dawley-Ratten durchgeführt. Als Kontrollgruppe diente eine scheinoperierte Gruppe, die operiert wurde, ohne dass die Riechkolben entfernt wurden. 14 Tage nach der Operation wurden die Ratten mit Vehikel, Rotigotin-Depotsuspension (jeden 2. Tag) oder Imipramin behandelt. An Testtagen wurden die Ratten auf ein Testfeld verbracht und für 3 Minuten sich selber überlassen. Dabei wurden die lokomotorischen Aktivitäten der Tiere anhand der Zahl überschrittener Linien gemessen.

## Patentansprüche

1. Arzneimittel umfassend Rotigotin [(-)-5,6,7,8-Tetrahydro-6-[propyl[2-(2-thienyl)ethyl]amino]-1-naphtol] oder ein Prodrug davon oder physiologisch akzeptable Salze davon zur Behandlung von Depressionen, wobei Prodrugs von Rotigotin Ester, Carbamate, Carbonate, Ketale, Acetate, Phospate, Phosphonate, Sulfate oder Sulfonate sind.

2. Arzneimittel nach Anspruch 1, wobei die affektive Störung ausgewählt ist aus depressiven Episoden, rezidivierenden depressiven Störungen, depressiven Phasen bei bipolaren affektiven Störungen, Angststörungen, Anpassungsstörungen und hirnorganischen Erkrankungen, die jeweils mit depressiven Symptomen einhergehen.

3. Arzneimittel umfassend Rotigotin [(-)-5,6,7,8-Tetrahydro-6-[propyl[2-(2-thienyl)ethyl]amino]-1-naphtol] oder ein Prodrug davon oder physiologisch akzeptable Salze davon zur Behandlung einer Depression, wobei Prodrugs von Rotigotin Ester, Carbamate, Carbonate, Ketale, Acetate, Phospate, Phosphonate, Sulfate oder Sulfonate sind und die Depression eine Morbus Parkinson-assoziierte Depression ist.

4. Arzneimittel nach einem der vorhergehenden Ansprüche, wobei das Arzneimittel zur parenteralen, transdermalen oder mukosalen Administration vorgesehen ist.

5. Arzneimittel nach einem der vorhergehenden Ansprüche, wobei das Rotigotin in einer Dosierung von 0,5 - 50 mg pro Tag verabreicht wird.

6. Arzneimittel nach Anspruch 1 oder 3, wobei das Arzneimittel ein Kombinationspräparat zur Behandlung von Depressionen umfassend einen weiteren Wirkstoff aus der Gruppe der Antidepressiva, Antipsychotika, Sedativa, Anxiolytika oder Migränemittel ist.

## Claims

1. Medicament comprising rotigotine [(-)-5,6,7,8-tetrahydro-6-[propyl[2-(2-thienyl)ethyl]amino]-1-naphthol] or a prodrug thereof or physiologically acceptable salts thereof for the treatment of depression, wherein prodrugs of rotigotine are esters, carbamates, carbonates, ketals, acetates, phosphates, phosphonates, sulphates or sulphonates.

2. Medicament according to claim 1, wherein the affective disturbance is selected from depressive episodes, recurring depressive disturbances, depressive phases in bipolar affective disturbances, anxiety disturbances, adaptation disturbances and cerebro-organic disorders which go along in each case with depressive symptoms.

3. Medicament comprising rotigotine [(-)-5,6,7,8-tetrahydro-6-[propyl[2-(2-thienyl)ethyl]amino]-1-naphthol] or a prodrug thereof or physiologically acceptable salts thereof for the treatment of depression, wherein prodrugs of rotigotine are esters, carbamates, carbonates, ketals, acetates, phosphates, phosphonates, sulphates or sulphonates and the depression is a Parkinson's disease-associated depression.

4. Medicament according to one of the preceding claims, wherein the medicament is provided for parenteral, transdermal or mucosal administration.

5. Medicament according to one of the preceding claims, wherein the rotigotine is administered in a dose of 0.5 - 50 mg per day.

6. Medicament according to claim 1 or 3, wherein the medicament is a combination preparation for the treatment of depression comprising a further active ingredient from the group of antidepressants, antipsychotics, sedatives, anxiolytics or anti-migraine preparations.

## Revendications

1. Médicament contenant de la rotigotine [(-)-5,6,7,8-tétrahydro-6-]propyl]2-(2-thiényl)éthyl]amino-1-naphto] ou un promédicament de celle-ci ou des sels physiologiquement acceptables de celle-ci pour le traitement des dépressions, les promédicaments de la rotigotine étant des esters, des carbamates, des carbonates, des cétals, des acétates, des phosphates, des phosphonates, des sulfates ou des sulfonates.

2. Médicament selon la revendication 1, dans lequel le trouble affectif est choisi parmi les épisodes dépressifs, les troubles dépressifs récidivants, les phases dépressives dans les troubles affectifs bipolaires, les troubles anxieux, les troubles de l'adaptation et les maladies cérébrales organiques, quand ceux-ci sont associés à des symptômes dépressifs.

3. Médicament contenant de la rotigotine [(-)-5,6,7,8-tétrahydro-6-[propyl]2-(2-thiényl)éthyl]amino-1-naphtol] ou un promédicament de celle-ci ou des sels physiologiquement acceptables de celle-ci pour le traitement des dépressions, les promédicaments de la rotigotine étant des esters, des carbamates, des carbonates, des cétals, des acétates, des phosphates, des phosphonates, des sulfates ou des sulfonates, et la dépression étant une dépression associée à la maladie de Parkinson.

4. Médicament selon l'une quelconque des revendications précédentes, lequel médicament est destiné à une administration par voie parentérale, transdermique ou muqueuse.

5. Médicament selon l'une quelconque des revendications précédentes, dans lequel la rotigotine est administrée à une dose de 0,5 à 50 mg par jour.

6. Médicament selon la revendication 1 ou 3, lequel médicament est une préparation combinée pour le traitement des dépressions contenant un autre principe actif faisant partie du groupe des antidépresseurs, des antipsychotiques, des sédatifs, des anxiolytiques ou des antimigraineux.
